# EUROPÄISCHE PATENTSCHRIFT

(11) **EP 2 997 509 B1**
(45) Veröffentlichungstag und Bekanntmachung des Hinweises auf die Patenterteilung: **07.07.2021**
(21) Anmeldenummer: 14724311.7
(22) Anmeldetag: 12.05.2014
(51) Int. Cl.: G16H 40/63, G16H 40/00

(54) **BILDSCHIRMANORDNUNG EINES MEDIZINISCHEN GERÄTS**
SCREEN ARRANGEMENT OF A MEDICAL APPLIANCE
APPAREIL MÉDICAL

(30) Priorität: 14.05.2013 DE 102013008213
(43) Veröffentlichungstag der Anmeldung: 23.03.2016
(73) Patentinhaber: Fresenius Medical Care Deutschland GmbH, 61352 Bad Homburg (DE)
(72) Erfinder: DORN, Vera, 63179 Obertshausen (DE); POHLMEIER, Robert, 61350 Bad Homburg (DE); WEHMEYER, Wolfgang, 72075 Tübingen (DE); GÖMPEL-KLEIN, Patricia, 61273 Wehrheim (DE); KRAUSE, Alfred, 61273 Wehrheim (DE)
(74) Vertreter: Herrmann, Uwe
(86) Internationale Anmeldenummer: PCT/EP2014/001278
(87) Internationale Veröffentlichungsnummer: WO 2014/183862

(56) Entgegenhaltungen:
- EP-A1- 1 859 720
- WO-A2-02/10847
- DE-A1- 2 810 216
- DE-U1-202010 010 770
- US-A1- 2002 152 045
- US-A1- 2012 185 267

## Beschreibung

Die vorliegende Erfindung betrifft ein medizinisches Gerät mit wenigstens einem Bildschirm, der wenigstens eine Fläche zur Anzeige von einer oder mehreren Informationen aufweist.

Derartige medizinische Geräte sind in zahlreichen unterschiedlichen Ausführungsformen aus dem Stand der Technik bekannt. Auf den Bildschirmen medizinischer Geräte werden Informationen, wie beispielsweise der allgemeine Betriebszustand des medizinischen Gerätes, d. h. die Information, ob das Gerät innerhalb normaler Parameter arbeitet oder ob gerade ein Alarmzustand oder Voralarmzustand eintritt, angezeigt. Des Weiteren sind sogenannte Alarmampeln bei medizinischen Geräten bekannt, die dem Nutzer des Gerätes unterschiedliche Alarmzustände signalisieren, um gegebenenfalls einen umgehenden Eingriff zu ermöglichen.

In einigen Bereichen, wie beispielsweise auf Intensivstationen stehen üblicherweise viele derartiger medizinischer Geräte auf engstem Raum. Die Bildschirme dieser Geräte sind aus ergonomischen Gründen häufig in einer ähnlichen Höhe angebracht, die sich an der Augenhöhe der Bediener orientiert.

Dies bringt den Nachteil mit sich, dass das Behandlungszimmer unübersichtlich wird und dass aufgrund der Mehrzahl von Bildschirmen gegebenenfalls der Blick auf den Patienten verdeckt wird. Zusätzlich kann der Fall eintreten, dass die Bildschirme sich gegenseitig verdecken, so dass gegebenenfalls Alarmmeldungen übersehen werden. Aufgrund der Vielzahl der Bildschirme und der darauf dargestellten Informationen kann es des Weiteren dazu kommen, dass wichtige Informationen nicht von unwichtigen Details differenziert werden können.

Auch wenn der Bildschirm eines einzelnen medizinischen Gerätes aufgrund seiner Größe groß dargestellte Informationen, die auch von der Ferne, beispielsweise von der Tür des Behandlungsraums aus lesbar sind, bereitstellt, besteht wie ausgeführt bei einer Mehrzahl derartiger Geräte der Nachteil, dass gegebenenfalls Informationen verdeckt werden und/oder es aufgrund der Vielzahl der sichtbaren Bildschirme schwer fällt, wesentliche Informationen zu identifizieren.

Der vorliegenden Erfindung liegt somit die Aufgabe zugrunde, ein medizinisches Gerät der eingangs genannten Art dahingehend weiterzubilden, dass die Übersichtlichkeit innerhalb eines Behandlungsbereichs auch bei einer Mehrzahl von medizinischen Geräten erhalten bleibt.

Diese Aufgabe wird durch ein medizinisches Gerät mit den Merkmalen des Anspruchs 1 und durch ein entsprechendes Verfahren nach Anspruch 7 gelöst. Weitere Ausführungsformen sind Gegenstand der abhängigen Ansprüche 2-6 und 8-11.

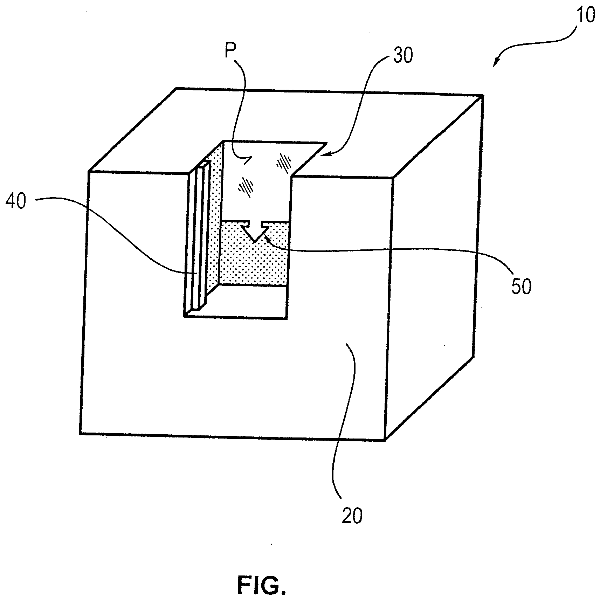

Einzelheiten und Vorteile der Erfindung werden anhand eines in der Zeichnung dargestellten Ausführungsbeispiels näher erläutert.

Die Figur zeigt eine schematische Darstellung eines medizinischen Gerätes mit einer Einsenkung und einem beleuchteten Teilbereich der Gehäuseoberfläche.

Das Bezugszeichen 10 kennzeichnet ein medizinisches Gerät, wie beispielsweise ein Blutbehandlungsgerät und insbesondere ein Dialysegerät, das einen Gerätekorpus mit einer Gehäuseoberfläche 20 aufweist. Innerhalb der Gehäuseoberfläche befindet sich eine Vertiefung, die in Form eines gegenüber der Frontseite des Gerätes zurückversetzten Abschnittes 30 ausgebildet ist.

Das Bezugszeichen 40 kennzeichnet eine LED-Leiste, die zur Beleuchtung der Projektionsfläche der Einsenkung 30 dient.

Um eine Information auf den Gerätekorpus oder einen Teilbereich von diesem wiederzugeben, sind Beleuchtungsmittel, beispielsweise in Form der dargestellten LED-Leiste 40 vorgesehen, die statisch oder intermittierend den gesamten Gerätekorpus oder wie in der Figur dargestellt einen Teilbereich des Gerätekorpus beleuchtet. Durch die Farbe bzw. Kontur der beleuchteten Fläche lässt sich eine Information 50 übermitteln.

Grundsätzlich können die folgenden Kategorien von Informationen unterschieden werden:
a) Alarmmeldung (kategorische Information "hohe Dringlichkeit für Anwenderinteraktion mit dem Gerät erforderlich" ohne Beschreibung der Details - z. B. bei Notstopp der Behandlung)
b) Warnmeldung (kategorische Information "mittlere Dringlichkeit für Anwenderinteraktion mit dem Gerät erforderlich" ohne Beschreibung von Details - z. B. bei Gerät wartet auf User-Input)
c) Trendmeldungen (mit graphischer Detailinformation - z. B. ein Füllstand eines Beutels)
d) deskriptive Detailinformationen (ausgedrückt in Buchstabe oder Zahl - z. B. Druck in mmHg).

Die Kategorien a) und b) werden traditionell über sogenannte Alarmampeln am medizinischen Gerät angezeigt, während für die Kategorien c) und d) ein Bildschirm verwendet wird.

Durch die Farbe und/oder Kontur der beleuchteten Fläche des Gerätekorpus kann beispielsweise eine Information gemäß a), b) oder c) übermittelt werden. Dabei können insbesondere die Farben rot, gelb und grün eingesetzt werden, um z. B. Alarme und Warnmeldungen entsprechend zu signalisieren. Vorzugsweise wird das Gerät insgesamt als Projektionsfläche für Informationen genutzt, was den Vorteil mit sich bringt, dass diese besonders gut erkennbar sind. Wie ausgeführt ist von der Erfindung jedoch auch der Fall umfasst, dass nur ein Teilbereich der äußeren Oberfläche des Gerätes als Projektionsfläche bzw. als beleuchtete Fläche genutzt wird.

Wie dies aus der Figur ersichtlich ist, kann die Gehäuseoberfläche eine oder mehrere Einsenkungen 30 aufweisen, von denen wenigstens eine Fläche, in dem hier dargestellten Ausführungsbeispiel die gegenüber der Außenseite zurückversetzte parallele Fläche der Einsenkung als Projektionsfläche P genutzt wird. Auf diese Weise lassen sich vergleichsweise große Symbole generieren, mit denen die Informationen nach a) bis c) transportiert werden.

Grundsätzlich ist die Erfindung jedoch nicht auf diese Informationen beschränkt, sondern umfasst beliebige Informationen, wie beispielsweise auch die deskriptiven Detailinformationen gemäß d).

Durch die in der Zeichnung dargestellte Ausführungsform ist es besonders gut möglich, eine Trendinformation nach Punkt c), d. h. beispielsweise eine Füllstandsinformation oder dergleichen durch eine variable Gestaltung des beleuchteten Bereichs darzustellen. Im Fall der in der Figur dargestellten rechteckigen Einsenkung einer senkrechten Gehäuseoberfläche kann beispielsweise der Füllstand eines Beutels symbolisiert werden, in dem durch eine LED-Leiste oder sonstige Leuchtmittel diese Fläche der Einsenkung entsprechend der Information über den aktuellen Zustand teilweise oder vollständig ausgeleuchtet wird.

## Patentansprüche

1. Medizinisches Gerät mit wenigstens einem Gerätegehäuse und mit wenigstens einer Fläche zur Anzeige von einer oder mehreren Informationen, wobei die Fläche zur Anzeige von Informationen durch einen Teilbereich des Gerätegehäuses gebildet wird, wobei Leuchtmittel vorgesehen sind, die derart ausgebildet sind, dass sie den Teilbereich des Gerätegehäuses beleuchten, und wobei vorgesehen ist, dass die Beleuchtung selbst die angezeigte Information oder einen Teil von dieser darstellt, **dadurch gekennzeichnet, dass** der Teilbereich in Form einer Einsenkung einer Oberfläche des Gerätegehäuses ausgestaltet ist und die Leuchtmittel als in der Einsenkung angebrachte LED-Leiste ausgestaltet sind, wobei die Einsenkung eine gegenüber der Außenseite des Gerätes zurückversetzte parallele Fläche aufweist, die als Projektionsfläche genutzt wird.

2. Medizinisches Gerät nach Anspruch 1, **dadurch gekennzeichnet, dass** die Information den Füllstand eines Kompartiments betrifft und die Fläche entsprechend der Information vollständig oder teilweise ausgeleuchtet wird.

3. Medizinisches Gerät nach Anspruch 1 oder 2, **dadurch gekennzeichnet, dass** die Information teilweise durch die Beleuchtungsfarbe gebildet wird.

4. Medizinisches Gerät nach einem der Ansprüche 1 bis 3, **dadurch gekennzeichnet, dass** die Fläche zur Anzeige von Informationen durch eine rechteckige Einsenkung des Gerätegehäuses gebildet wird.

5. Medizinisches Gerät nach einem der Ansprüche 1 bis 4, **dadurch gekennzeichnet, dass** die Leuchtmittel organische LEDs umfassen.

6. Medizinisches Gerät nach einem der Ansprüche 1 bis 7, **dadurch gekennzeichnet, dass** das Kompartiment ein an das medizinische Gerät angeschlossener Beutel ist.

7. Verfahren zum Anzeigen von einer oder mehreren Informationen an einem medizinischen Gerät, wobei die Informationen auf einem Teilbereich des Gerätegehäuses angezeigt werden und wobei zur Anzeige der Informationen Leuchtmittel in Form einer LED-Leiste verwendet werden, die den Teilbereich des Gerätegehäuses beleuchten, wobei vorgesehen ist, dass die Beleuchtung selbst die angezeigte Information oder einen Teil von dieser darstellt, **dadurch gekennzeichnet, dass** der Teilbereich in Form einer Einsenkung einer Oberfläche des Gerätegehäuses ausgestaltet und die Leuchtmittel als in der Einsenkung angebrachte LED-Leiste ausgestaltet sind, wobei die Einsenkung eine gegenüber der Außenseite des Gerätes zurückversetzte parallele Fläche aufweist, die als Projektionsfläche genutzt wird.

8. Verfahren nach Anspruch 7, **dadurch gekennzeichnet, dass** die Information den Füllstand eines Kompartiments betrifft und die Fläche entsprechend der Information vollständig oder teilweise ausgeleuchtet wird.

9. Verfahren nach Anspruch nach Anspruch 7 oder 8, **dadurch gekennzeichnet, dass** die Informationen teilweise durch die Beleuchtungsfarbe dargestellt werden.

10. Verfahren nach einem der Ansprüche 7 bis 9 **dadurch gekennzeichnet, dass** es sich bei dem medizinischen Gerät um ein medizinisches Gerät nach einem der Ansprüche 1 bis 8 handelt.

11. Verfahren nach einem der Ansprüche 7 bis 10, **dadurch gekennzeichnet, dass** das Kompartiment ein an das medizinische Gerät angeschlossener Beutel ist.

## Claims

1. A medical device having at least one device housing and having at least one area for displaying one or more pieces of information, wherein the area for displaying information is formed by a partial region of the device housing, wherein lamps are provided which are configured such that they illuminate the partial region of the device housing, and wherein provision is made that the illumination itself represents the displayed information or a part thereof, **characterized in that** the partial region is configured in the form of a depression of a surface of the device housing and the lamps are configured as an LED strip attached in the depression, with the depression having a parallel surface that is set back with respect to the outer side of the device and that is used as a projection surface.

2. A medical device in accordance with claim 1, **characterized in that** the information relates to the filling level of a compartment and the area corresponding to the information is illuminated in part or in full.

3. A medical device in accordance with claim 1 or claim 2, **characterized in that** the information is partially formed by the illumination color

4. A medical device in accordance with one of the claims 1 to 3, **characterized in that** the area for the display of information is formed by a rectangular depression of the device housing.

5. A medical device in accordance with one of the claims 1 to 4, **characterized in that** the lamps comprise organic LEDs.

6. A medical device in accordance with one of the claims 1 to 5, **characterized in that** the compartment is a bag connected to the medical device.

7. A method of displaying one or more pieces of information at a medical device, wherein the information is displayed on a partial region of the device housing and wherein lamps in the form of an LED strip are used to display the information, and wherein provision is made that the illumination itself represents the displayed information or a part thereof, **characterized in that** the partial region is configured in the form of a depression of a surface of the device housing and the lamps are configured as an LED strip attached in the depression, with the depression having a parallel surface that is set back with respect to the outer side of the device and that is used as a projection surface.

8. A method in accordance with claim 7, **characterized in that** the information relates to the filling level of a compartment and the area corresponding to the information is illuminated in part or in full.

9. A method in accordance with claim 7 or claim 8, **characterized in that** the information is partially formed by the illumination color

10. A method in accordance with one of the claims 7 to 9, **characterized in that** the medical device is a medical device in accordance with one of the claims 1 to 8.

11. A method in accordance with one of the claims 7 to 10, **characterized in that** the compartment is a bag connected to the medical device.

## Revendications

1. Appareil médical comprenant au moins un boîtier d'appareil et au moins une surface destinée à l'affichage d'une ou de plusieurs informations, la surface destinée à l'affichage d'informations étant formée par une partie du boîtier d'appareil, des moyens d'éclairage réalisés de manière à éclairer la partie du boîtier d'appareil étant prévus, et l'éclairage étant prévu pour représenter lui-même l'information affichée ou une partie de celle-ci, **caractérisé en ce que** la partie est conçue sous la forme d'un renfoncement d'une surface du boîtier d'appareil et les moyens d'éclairage sont conçus sous la forme d'une barre à DEL placée dans le renfoncement, le renfoncement comportant une surface parallèle en retrait par rapport au côté extérieur de l'appareil, ladite surface étant utilisée comme surface de projection.

2. Appareil médical selon la revendication 1, **caractérisé en ce que** l'information concerne le niveau de remplissage d'un compartiment et la surface est éclairée entièrement ou partiellement en fonction de l'information.

3. Appareil médical selon la revendication 1 ou 2, **caractérisé en ce que** l'information est formée en partie par la couleur d'éclairage.

4. Appareil médical selon l'une des revendications 1 à 3, **caractérisé en ce que** la surface destinée à l'affichage d'informations est formée par un renfoncement rectangulaire du boîtier d'appareil.

5. Appareil médical selon l'une des revendications 1 à 4, **caractérisé en ce que** les moyens d'éclairage comprennent des DEL organiques.

6. Appareil médical selon l'une des revendications 1 à 7, **caractérisé en ce que** le compartiment est une poche reliée à l'appareil médical.

7. Procédé d'affichage d'une ou de plusieurs informations sur un appareil médical, les informations étant affichées sur une partie du boîtier d'appareil et des moyens d'éclairage sous la forme d'une barre à DEL étant utilisés pour l'affichage des informations, lesdits moyens d'éclairage éclairant la partie du boîtier d'appareil, l'éclairage étant prévu pour représenter lui-même l'information affichée ou une partie de celle-ci, **caractérisé en ce que** la partie est conçue sous la forme d'un renfoncement d'une surface du boîtier d'appareil et les moyens d'éclairage sont conçus sous la forme d'une barre à DEL placée dans le renfoncement, le renfoncement comportant une surface parallèle en retrait par rapport au côté extérieur de l'appareil, ladite surface étant utilisée comme surface de projection.

8. Procédé selon la revendication 7, **caractérisé en ce que** l'information concerne le niveau de remplissage d'un compartiment et la surface est éclairée entièrement ou partiellement en fonction de l'information.

9. Procédé selon la revendication 7 ou 8, **caractérisé en ce que** les informations sont représentées en partie par la couleur d'éclairage.

10. Procédé selon l'une des revendications 7 à 9, **caractérisé en ce que** l'appareil médical est un appareil médical selon l'une des revendications 1 à 8.

11. Procédé selon l'une des revendications 7 à 10, **caractérisé en ce que** le compartiment est une poche reliée à l'appareil médical.
